# EUROPEAN PATENT APPLICATION

(11) **EP 4 516 918 A1**
(43) Date of publication of application: **05.03.2025**
(21) Application number: 23787612.3
(22) Date of filing: 07.04.2023
(51) Int. Cl.: C12P 13/00, C12N 15/70, C12N 1/21, C12N 15/53, C12R 1/19

(54) **METHOD FOR PREPARING ISOPROPANOLAMINE**

(30) Priority: 11.04.2022 CN 202210371284
(71) Applicant: Mint Biotechnologies Co., Ltd., Hangzhou, Zhejiang 310012 (CN)
(72) Inventor: CAO, Chenkai, Hangzhou, Zhejiang 310012 (CN); ZHANG, Kechun, Hangzhou, Zhejiang 310012 (CN)
(74) Representative: Gille Hrabal Partnerschaftsgesellschaft mbB Patentanwälte
(86) International application number: PCT/CN2023/087079
(87) International publication number: WO 2023/197968

(57) **Abstract**

The present invention relates to the technical field of genetic engineering and fermentation engineering, and particularly, to a method for preparing isopropanolamine. The method is characterized in that threonine is converted into L-2-amino-3-oxobutyric acid under the action of an oxidase, L-2-amino-3-oxobutyric acid is decarboxylated spontaneously to give aminoacetone, and the aminoacetone is converted into isopropanolamine under the action of a reductase, the isopropanolamine including 1-amino-(R)-2-propanol or 1-amino-(S)-2-propanol.

## Description

The present application claims priority to Chinese Patent Application No. 202210371284.X filed to China National Intellectual Property Administration on Apr. 11, 2022, and entitled "METHOD FOR PREPARING ISOPROPANOLAMINE", which is incorporated herein by reference in its entirety.

### TECHNICAL FIELD

The present disclosure relates to the technical field of genetic engineering and fermentation engineering, and particularly relates to a preparation method for isopropanolamine.

### BACKGROUND

Isopropanolamine is an important basic chemical starting material, widely applied in the fields of metal protective agents, acid gas absorbents, cement grinding aids, etc., and can be used as a refining agent, antistatic agent, coloring agent, and fiber wetting agent in the fiber industry. It can also be used for synthesizing detergents, and can be used for preparing antioxidants, plasticizers, emulsifiers, and solvents of cosmetic lubricating oil and cutting oil.

The current methods for producing isopropanolamine include a propylene oxide method, a calcium cyanamide method, and a supercritical fluid method. For the propylene oxide method, propylene oxide and ammonia are mixed, and then preheated to perform a ring opening reaction to produce a mixture. The mixture is then dehydrogenated, dehydrated, distilled under reduced pressure, and rectified to give isopropanolamine. In the process, diisopropanolamine and triisopropanolamine can also be produced depending on the feeding ratio of the propylene oxide and the ammonia. For the calcium cyanamide method, 2-amino-3-(2-hydroxypropyl)-5-methyl-1,3-dioxazolidine and calcium carbonate are produced under the action of propylene oxide and calcium cyanamide. The oxazolidine produces diisopropanolamine in the presence of potassium hydroxide or potassium carbonate. For the supercritical fluid method, ammonia and propylene oxide are used as starting materials, and water is used as a catalyst. Isopropanolamine is continuously produced by processes of flash evaporation, two-step absorption deamination and continuous rectification under reduced pressure, falling-film evaporation, and the like in a supercritical state.

The synthesis methods described above have the problems of involving complicated steps, environmentally unfriendly starting materials and intermediate products, use of highly toxic cyanide, harsh reaction conditions, complicated by-products, low transformation rate, low yield, and the like, and need to be improved urgently.

### SUMMARY

The technical problem to be solved by the present disclosure is to provide a preparation method for isopropanolamine, which is more applicable for industrial production and more environmentally friendly with a higher yield.

The present disclosure provides a preparation method for isopropanolamine, wherein threonine is converted into isopropanolamine in the presence of an oxidase and a reductase, and the isopropanolamine is selected from 1-amino-(R)-2-propanol and/or 1-amino-(S)-2-propanol, and is preferably 1-amino-(S)-2-propanol.

The present disclosure further provides a preparation method for isopropanolamine, wherein threonine is converted into L-2-amino-3-oxobutyric acid in the presence of an oxidase, the L-2-amino-3-oxobutyric acid is spontaneously decarboxylated to give aminoacetone, and the aminoacetone is converted into isopropanolamine in the presence of a reductase; the isopropanolamine comprises 1-amino-(R)-2-propanol or 1-amino-(S)-2-propanol.

In some embodiments, the conversion is completed within a bacterial or fungal organism.

In some embodiments, the threonine is used as a substrate, a recombinant microorganism comprising an oxidase-encoding gene and a reductase-encoding gene is added for fermentation culture, and during the fermentation, the oxidase and the reductase are produced by overexpression of the recombinant microorganism.

In some embodiments, the oxidase-encoding gene comprises any one or more of tdh, yiaY, and adhB, and is preferably tdh; more preferably, the nucleotide sequence of the tdh gene is set forth in SEQ ID NO: 1.

In some embodiments, the reductase-encoding gene comprises any one or more of gre2p, egsA, SU7, and VIN7, and is preferably gre2p; more preferably, the nucleotide sequence of the gre2p gene is set forth in SEQ ID NO: 2.

In some embodiments, the preparation method further comprises constructing the recombinant microorganism by a genetic engineering method, wherein the genetic engineering method preferably comprises plasmid expression or genomic integration.

In some embodiments, the recombinant microorganism is constructed by the plasmid expression method, and the construction method is as follows: an oxidase-encoding gene and a reductase-encoding gene are obtained by PCR amplification, the obtained genes are co-ligated to a plasmid vector (e.g., a plasmid vector comprising an IPTG inducible promoter, preferably a vector pZElac comprising an IPTG inducible promoter), and transformed into a competent cell (e.g., an *Escherichia coli* dh5a competent cell), and after sequencing, a recombinant vector is obtained; and the recombinant vector is transformed into a microorganism to give the recombinant microorganism.

In some embodiments, the recombinant vector is pZE-tdh-gre2p; preferably, a construction method for the pZE-tdh-gre2p is as follows: a tdh gene and a gre2p gene are obtained by PCR amplification, and the tdh gene and the gre2p gene are co-ligated to the vector pZElac comprising an IPTG inducible promoter and transformed to a competent cell; and after sequencing, the plasmid pZE-tdh-gre2p is obtained; a construction method for the pZE-tdh-gre2p is illustratively as follows: a tdh gene and a gre2p gene are separately obtained by PCR amplification by using the genomes of *Escherichia coli* MG1655 and *Saccharomyces cerevisiae* S288C as templates, and the tdh gene and the gre2p gene are co-ligated to the vector pZElac comprising an IPTG inducible promoter, and transformed into an *Escherichia coli* dh5a competent cell; and after sequencing, the plasmid pZE-tdh-gre2p is obtained.

In some embodiments, the microorganism is selected from one or more of *Escherichia coli, Bacillus, Corynebacterium, Saccharomyces,* or *Streptomyces.*

In some embodiments, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

In some embodiments, during the fermentation, the fermentation temperature is 20-90 °C.

In some embodiments, during the fermentation, a culture medium adopted comprises starting materials in the following proportions: 11-13 g/L of M9 salts, 1-5 g/L of magnesium sulfate, 0.1-0.5 g/L of calcium chloride, 0.01-0.05 g/L of thiamine, 10-100 g/L of auxiliary material, 3-8 g/L of yeast powder, 1-3 mM/L of IPTG, and 30-60 µg/mL of ampicillin.

In some embodiments, the auxiliary material is D-glucose.

The present disclosure further provides a recombinant microorganism for preparing isopropanolamine, wherein the recombinant microorganism comprises an oxidase-encoding gene and a reductase-encoding gene;
preferably, the oxidase-encoding gene comprises any one or more of tdh, yiaY, and adhB, and is preferably tdh; more preferably, the nucleotide sequence of the tdh gene is set forth in SEQ ID NO: 1;
and/or, the reductase-encoding gene comprises any one or more of gre2p, egsA, SU7, and VIN7, and is preferably gre2p; more preferably, the nucleotide sequence of the gre2p gene is set forth in SEQ ID NO: 2;
preferably, the microorganism is selected from one or more of *Escherichia coli, Bacillus, Corynebacterium, Saccharomyces,* or *Streptomyces.* More preferably, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

The present disclosure further provides a recombinant DNA or biomaterial for preparing isopropanolamine, wherein the recombinant microorganism or biomaterial comprises an oxidase-encoding gene and a reductase-encoding gene;
preferably, the oxidase-encoding gene comprises any one or more of tdh, yiaY, and adhB, and is preferably tdh; more preferably, the nucleotide sequence of the tdh gene is set forth in SEQ ID NO: 1;
and/or, the reductase-encoding gene comprises any one or more of gre2p, egsA, SU7, and VIN7, and is preferably gre2p; more preferably, the nucleotide sequence of the gre2p gene is set forth in SEQ ID NO: 2;
preferably, the biomaterial is an expression cassette, a transposon, a plasmid vector, a phage vector, or a virus vector.

The present disclosure further provides use of the recombinant microorganism described above and the recombinant DNA or biomaterial described above for manufacturing isopropanolamine.

Beneficial effects: According to the preparation method for isopropanolamine described herein, threonine is oxidized into L-2-amino-3-oxobutyric acid by using an enzyme, the obtained L-2-amino-3-oxobutyric acid is spontaneously decarboxylated to give aminoacetone, and the aminoacetone is reduced into isopropanolamine by using a reductase. According to the method of the present disclosure, low-cost starting materials are used, and the preparation process is simple and environmentally friendly with mild operation means, which avoids the use of highly toxic cyanide. With a high transformation rate and yield, it is applicable for industrial processing and production.

According to the present disclosure, isopropanolamine with two different configurations can be produced in a mild environment. It has good production popularization and application value.

### DETAILED DESCRIPTION

In order to make the technical means, creation characteristics, achieved purposes, and effects of the present disclosure easy to understand, the present disclosure is further illustrated below with reference to specific examples.

In the present disclosure, unless otherwise indicated, scientific and technical terms have the same meaning as commonly understood by those skilled in the art. In addition, the terms and laboratory procedures related to nucleic acid chemistry, molecular biology, cell and tissue culture, microbiology, and immunology used herein are the terms and conventional procedures widely used in the corresponding fields. Also, in order to better understand the present disclosure, the definitions and interpretations of the related terms are provided below.

It should be appreciated that the terms used herein are for the purpose of illustrating particular embodiments only, and are not intended to be limiting.

The articles "a", "an" and "the" are used herein to refer to one or more than one of the grammatical object of the article.

The use of alternatives (e.g., "or") should be understood to refer to one, two, or any combination of the alternatives. The term "and/or" should be interpreted as referring to one or both of the alternatives.

As used herein, the term "gene synthesis" refers to a generation process using recombinant DNA techniques or a production process using DNA or amino acid sequence synthesis techniques available and well known in the art.

The term "encode" or "code" refers to the inherent property of specific sequences of nucleotides in a polynucleotide, such as a gene, a cDNA, or an mRNA, to serve as a template in synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of an mRNA corresponding to the gene produces the protein in a cell or other biological system. Both the coding strand, which comprises a nucleotide sequence equivalent to the mRNA sequence and is usually provided in a sequence listing, and the non-coding strand, which is used as a template for transcription of a gene or cDNA, may be referred to as encoding the protein or other product of that gene or cDNA.

As used herein, the term "endogenous" refers to any substance derived from or produced within an organism, cell, tissue or system.

As used herein, the term "exogenous" refers to any substance introduced into or produced outside of an organism, cell, tissue or system.

As used herein, the term "expression" is defined as the transcription and/or translation of a particular nucleotide sequence driven by its promoter.

Unless otherwise specified, the "polynucleotide sequence encoding an amino acid sequence" includes all nucleotide sequences that are degenerate versions of each other and encode the same amino acid sequence. The phrase "nucleotide sequence encoding a protein or an RNA" may also include introns to the extent that the nucleotide sequence encoding the protein may contain an intron(s) in some versions.

As used herein, the term "vector" refers to a composition of matter that comprises an isolated nucleic acid and can be used to deliver the isolated nucleic acid to the interior of a cell. The transferred nucleic acid is typically ligated to, e.g., inserted into, a vector nucleic acid molecule. A vector may comprise sequences that direct autonomous replication in the cell, or may comprise sequences sufficient to allow integration into the host cell DNA. Many vectors are known in the art, including but not limited to plasmids, phagemids, artificial chromosomes, bacteriophages and animal viruses. Therefore, the term "vector" encompasses an autonomously replicating plasmid or virus.

The DNA polymerase Phanta Max Super-Fidelity DNA Polymerase and the non-ligase-dependent single-fragment quick cloning kit ClonExpress^{®}II One Step used in the embodiments of the present disclosure are purchased from Nanjing Vazyme Biotech Co., Ltd.

LB medium composition: 10 g/L of tryptone, 5 g/L of yeast powder, 10 g/L of sodium chloride, and 1.5% of agar powder added into a solid culture medium.

Antibiotic concentration: 50 µg/mL of ampicillin.

The detection method for isopropanolamine: the quantitative detecting of isopropanolamine by using an HPLC-RID equipped with the Agilent chromatographic column (Agilent InfinityLab Poroshell 120 Columns).

The recombinant vectors used in Examples 1 and 2 are constructed as follows.

Primers were designed separately based on the genomic sequences of *Escherichia coli* MG1655 and *Saccharomyces cerevisiae* S288C published by NCBI:

| | |
|---|---|
| PZE_Nhe1_F | CGCGCTAGCTCTAGAGGCATCAAATAAAACGAAAGGC |
| PZE_Kpn1_R | CGGGGTACCTTTCTCCTCTTTAATGAATTCG |
| tdh_kpn1_F | CGGGGTACCATGAAAGCGTTATCCAAACTGAAAG |
| tdh_BamH1_R | CGCGGATCCTTAATCCCAGCTCAGAATAACTTTC |
| gre2p_BamH1_F | CGCggatccAGGAGAAATTAACTATGTCAGTTTTCGTTTCAGGTGCTA ACGGG |
| gre2p_Nhe1_R | CGCGCTAGCTTATATTCTGCCCTCAAATT |
| gldA _BamH1_F | CGCGGATCCAGGAGAAATTAACTATGGACCGCATTATTCAATCAC CGGGTAAA |
| gldA _Nhe1_R | CGCgctagcTTATTCCCACTCTTGCAGGA |

A tdh gene fragment was obtained by PCR amplification by using the genome of *Escherichia coli* MG1655 as a template, and a gre2p gene fragment was obtained by PCR amplification by using the genome of *Saccharomyces cerevisiae* S288C as a template. The genes were ligated to a vector pZElac comprising an IPTG inducible promoter by the non-ligase-dependent single-fragment quick cloning kit, and then transformed into BW25113 competent cells. A kanamycin sulfate resistance plate was coated with the cells for culturing overnight. Positive clones were selected for sequencing verification, and the correct recombinant vector was designated as pZE-tdh-gre2p.

A tdh gene fragment and a gldA gene fragment were obtained by PCR amplification by using the genome of *Escherichia coli* MG1655 as a template. The genes were ligated to a vector pZElac comprising an IPTG inducible promoter by the non-ligase-dependent single-fragment quick cloning kit, and then transformed into BW25113 competent cells. A kanamycin sulfate resistance plate was coated with the cells for culturing overnight. Positive clones were selected for sequencing verification, and the correct recombinant vector was designated as pZE-tdh-gldA.

Wherein, the nucleotide sequence of the tdh gene is set forth in SEQ ID NO: 1.

The nucleotide sequence of the gre2p gene is set forth in SEQ ID NO: 2.

The nucleotide sequence of the gldA gene is set forth in SEQ ID NO: 3.

### Example 1

The recombinant vector pZE-tdh-gre2p was transformed into *Escherichia coli* BW25113 to give recombinant bacteria, and the single colonies of the recombinant bacteria described above were separately seeded into a 2 mL LB liquid medium containing 50 µg/mL ampicillin. The mixture was cultured at 37 °C and 220 rpm overnight (about 14 h). The bacteria were transferred to a 100 mL Erlenmeyer flask containing 10 mL fermentation medium at an initial OD value of 0.05, and threonine, as a substrate, was added to the fermentation medium at a ratio of 8.57 g/L (the ratio of threonine to the fermentation medium). The mixture was cultured at 30 °C and 220 rpm. To each fermentation flask was added 0.5 g CaCO₃ to adjust the pH of the fermentation broth. After 12 h and 24 h of culture, the fermentation broth was collected, and the concentrations of threonine and isopropanolamine were detected (see Table 1). The threonine was converted to L-2-amino-3-oxobutyric acid in the presence of an oxidase encoded by tdh, the L-2-amino-3-oxobutyric acid was spontaneously decarboxylated to give aminoacetone, and the aminoacetone was converted into isopropanolamine in the presence of a reductase encoded by gre2p.

The fermentation medium used was as follows:

| Component | Concentration |
|---|---|
| M9 salts | 11.2 g/L |
| Magnesium sulfate | 1 g/L |
| Calcium chloride | 0.1 g/L |
| Thiamine | 0.01 g/L |
| D-glucose | 40 g/L |
| Yeast powder | 5 g/L |
| IPTG | 1 mM/L |
| Ampicillin | 50 µg/mL |

### Example 2

The example is a reference example. The recombinant vector pZE-tdh-gldA was transformed into *Escherichia coli* BW25113 to give a recombinant bacteria, and the single colonies of the recombinant bacteria described above were separately seeded into a 2 mL LB liquid medium containing 50 µg/mL ampicillin. The mixture was cultured at 37 °C and 220 rpm overnight (about 14 h). The bacteria were transferred to a 100 mL Erlenmeyer flask containing 10 mL fermentation medium at an initial OD value of 0.05, and threonine, as a substrate, was added to the fermentation medium at a ratio of 8.57 g/L (the ratio of threonine to the fermentation medium). The mixture was cultured at 32 °C and 220 rpm. To each fermentation flask was added 0.5 g CaCO₃ to adjust the pH of the fermentation broth. After 12 h and 24 h of culture, the fermentation broth was collected, and the concentrations of threonine and isopropanolamine were detected (see Table 1).

The detection results of Examples 1 and 2 are shown in Table 1. It can be seen that, in Example 1, after 12 h of fermentation, the concentration of isopropanolamine in the fermentation broth was 0.43 g/L, with a transformation rate of 22.6%, and after 24 h of fermentation, the concentration of isopropanolamine in the fermentation broth was up to 1.48 g/L, with a transformation rate of 43.8%. In the example, all the obtained isopropanolamine was S-configuration isopropanolamine, while in some other examples, the configuration of isopropanolamine products can be controlled by selecting enzyme genes. However, in Example 2, certain threonine was converted in the fermentation process due to the presence of the oxidase, while gldA, which is also a reductase-encoding gene, could not catalyze the production of isopropanolamine.

**Table 1 Detection result table**

| | Time/h | Threonine/g·L⁻¹ | Isopropanolamine/g·L⁻¹ |
|---|---|---|---|
| pZE-tdh-gre2p | 12 | 5.55 | 0.43 |
| | 24 | 3.21 | 1.48 |
| pZE-tdh-gldA | 12 | 7.13 | Undetectable |
| | 24 | 6.25 | Undetectable |

The foregoing shows and describes the general principles, principal features, and advantages of the present disclosure. It should be understood by those skilled in the art that the present disclosure is not limited to the examples described above, and the examples described above and the description in the specification are merely illustration of the principles of the present disclosure. Various changes and modifications may be made without departing from the spirit and scope of the present disclosure, and such changes and modifications are within the protection scope of the present disclosure as claimed. The protection scope of the present disclosure as claimed is defined by the appended claims and equivalents thereof.

### Sequence information:

SEQ ID: 1
   Length: 1026
   Type: DNA
   Species: *Escherichia coli* MG1655 
atgaaagcgt tatccaaact gaaagcggaa gagggcatct ggatgaccga cgttcctgta 60

ccggaactcg ggcataacga tctgctgatt aaaatccgta aaacagccat ctgcgggact 120

gacgttcaca tctataactg ggatgagtgg tcgcaaaaaa ccatcccggt gccgatggtc 180

gtgggccatg aatatgtcgg tgaagtggta ggtattggtc aggaagtgaa aggcttcaag 240

atcggcgatc gcgtttctgg cgaaggccat atcacctgtg gtcattgccg caactgtcgt 300

ggtggtcgta cccatttgtg ccgcaacacg ataggcgttg gtgttaatcg cccgggctgc 360

tttgccgaat atctggtgat cccggcattc aacgccttca aaatccccga caatatttcc 420

gatgacttag ccgcaatttt tgatcccttc ggtaacgccg tgcataccgc gctgtcgttt 480

gatctggtgg gcgaagatgt gctggtttct ggtgcaggcc cgattggtat tatggcagcg 540

gcggtggcga aacacgttgg tgcacgcaat gtggtgatca ctgatgttaa cgaataccgc 600

cttgagctgg cgcgtaaaat gggtatcacc cgtgcggtta acgtcgccaa agaaaatctc 660

aatgacgtga tggcggagtt aggcatgacc gaaggttttg atgtcggtct ggaaatgtcc 720

ggtgcgccgc cagcgtttcg taccatgctt gacaccatga atcacggcgg ccgtattgcg 780
 
atgctgggta ttccgccgtc tgatatgtct atcgactgga ccaaagtgat ctttaaaggc 840

ttgttcatta aaggtattta cggtcgtgag atgtttgaaa cctggtacaa gatggcggcg 900

ctgattcagt ctggcctcga tctttcgccg atcattaccc atcgtttctc tatcgatgat 960

ttccagaagg gctttgacgc tatgcgttcg ggccagtccg ggaaagttat tctgagctgg 1020

gattaa 1026
SEQ ID: 2
   Length: 1029
   Type: DNA
   Species: *Saccharomyces cerevisiae* S288C 
atgtcagttt tcgtttcagg tgctaacggg ttcattgccc aacacattgt cgatctcctg 60

ttgaaggaag actataaggt catcggttct gccagaagtc aagaaaaggc cgagaattta 120

acggaggcct ttggtaacaa cccaaaattc tccatggaag ttgtcccaga catatctaag 180

ctggacgcat ttgaccatgt tttccaaaag cacggcaagg atatcaagat agttctacat 240

acggcctctc cattctgctt tgatatcact gacagtgaac gcgatttatt aattcctgct 300

gtgaacggtg ttaagggaat tctccactca attaaaaaat acgccgctga ttctgtagaa 360

cgtgtagttc tcacctcttc ttatgcagct gtgttcgata tggcaaaaga aaacgataag 420

tctttaacat ttaacgaaga atcctggaac ccagctacct gggagagttg ccaaagtgac 480

ccagttaacg cctactgtgg ttctaagaag tttgctgaaa aagcagcttg ggaatttcta 540
 
gaggagaata gagactctgt aaaattcgaa ttaactgccg ttaacccagt ttacgttttt 600

ggtccgcaaa tgtttgacaa agatgtgaaa aaacacttga acacatcttg cgaactcgtc 660

aacagcttga tgcatttatc accagaggac aagataccgg aactatttgg tggatacatt 720

gatgttcgtg atgttgcaaa ggctcattta gttgccttcc aaaagaggga aacaattggt 780

caaagactaa tcgtatcgga ggccagattt actatgcagg atgttctcga tatccttaac 840

gaagacttcc ctgttctaaa aggcaatatt ccagtgggga aaccaggttc tggtgctacc 900

cataacaccc ttggtgctac tcttgataat aaaaagagta agaaattgtt aggtttcaag 960

ttcaggaact tgaaagagac cattgacgac actgcctccc aaattttaaa atttgagggc 1020

agaatataa 1029
SEQ ID: 3
   Length: 1104
   Type: DNA
   Species: *Escherichia coli* MG1655

atggaccgca ttattcaatc accgggtaaa tacatccagg gcgctgatgt gattaatcgt 60

ctgggcgaat acctgaagcc gctggcagaa cgctggttag tggtgggtga caaatttgtt 120

ttaggttttg ctcaatccac tgtcgagaaa agctttaaag atgctggact ggtagtagaa 180

attgcgccgt ttggcggtga atgttcgcaa aatgagatcg accgtctgcg tggcatcgcg 240

gagactgcgc agtgtggcgc aattctcggt atcggtggcg gaaaaaccct cgatactgcc 300

aaagcactgg cacatttcat gggtgttccg gtagcgatcg caccgactat cgcctctacc 360

gatgcaccgt gcagcgcatt gtctgttatc tacaccgatg agggtgagtt tgaccgctat 420

ctgctgttgc caaataaccc gaatatggtc attgtcgaca ccaaaatcgt cgctggcgca 480

cctgcacgtc tgttagcggc gggtatcggc gatgcgctgg caacctggtt tgaagcgcgt 540

gcctgctctc gtagcggcgc gaccaccatg gcgggcggca agtgcaccca ggctgcgctg 600

gcactggctg aactgtgcta caacaccctg ctggaagaag gcgaaaaagc gatgcttgct 660

gccgaacagc atgtagtgac tccggcgctg gagcgcgtga ttgaagcgaa cacctatttg 720

agcggtgttg gttttgaaag tggtggtctg gctgcggcgc acgcagtgca taacggcctg 780

accgctatcc cggacgcgca tcactattat cacggtgaaa aagtggcatt cggtacgctg 840

acgcagctgg ttctggaaaa tgcgccggtg gaggaaatcg aaaccgtagc tgcccttagc 900

catgcggtag gtttgccaat aactctcgct caactggata ttaaagaaga tgtcccggcg 960

aaaatgcgaa ttgtggcaga agcggcatgt gcagaaggtg aaaccattca caacatgcct 1020

ggcggcgcga cgccagatca ggtttacgcc gctctgctgg tagccgacca gtacggtcag 1080

cgtttcctgc aagagtggga ataa 1104

## Claims

1. A preparation method for isopropanolamine, wherein threonine is converted into isopropanolamine in the presence of an oxidase and a reductase, and the isopropanolamine is selected from 1-amino-(R)-2-propanol and/or 1-amino-(S)-2-propanol, and is preferably 1-amino-(S)-2-propanol.

2. A preparation method for isopropanolamine, wherein threonine is converted into L-2-amino-3-oxobutyric acid in the presence of an oxidase, the L-2-amino-3-oxobutyric acid is spontaneously decarboxylated to give aminoacetone, and the aminoacetone is converted into isopropanolamine in the presence of a reductase; the isopropanolamine comprises 1-amino-(R)-2-propanol or 1-amino-(S)-2-propanol.

3. The preparation method for isopropanolamine as claimed in claim 1 or 2, wherein the conversion is completed within a bacterial or fungal organism.

4. The preparation method for isopropanolamine as claimed in claims 1-3, wherein the threonine is used as a substrate, a recombinant microorganism comprising an oxidase-encoding gene and a reductase-encoding gene is added for fermentation culture, and during the fermentation, the oxidase and the reductase are produced by overexpression of the recombinant microorganism.

5. The preparation method for isopropanolamine as claimed in any one of claims 1-4, wherein the oxidase-encoding gene comprises any one or more of tdh, yiaY, and adhB, and is preferably tdh; more preferably, the nucleotide sequence of the tdh gene is set forth in SEQ ID NO: 1;
and/or, the reductase-encoding gene comprises any one or more of gre2p, egsA, SU7, and VIN7, and is preferably gre2p; more preferably, the nucleotide sequence of the gre2p gene is set forth in SEQ ID NO: 2.

6. The preparation method for isopropanolamine as claimed in claim 4 or 5, wherein the preparation method further comprises constructing the recombinant microorganism by a genetic engineering method, wherein the genetic engineering method preferably comprises plasmid expression or genomic integration.

7. The preparation method for isopropanolamine as claimed in claim 6, wherein the recombinant microorganism is constructed by the plasmid expression method, and the construction method is as follows: an oxidase-encoding gene and a reductase-encoding gene are obtained by PCR amplification, the obtained genes are co-ligated to a plasmid vector (e.g., a plasmid vector comprising an IPTG inducible promoter, preferably a vector pZElac comprising an IPTG inducible promoter), and transformed into a competent cell (e.g., an *Escherichia coli* dh5a competent cell), and after sequencing, a recombinant vector is obtained; and the recombinant vector is transformed into a microorganism to give the recombinant microorganism.

8. The preparation method for isopropanolamine as claimed in claim 7, wherein the recombinant vector is pZE-tdh-gre2p; preferably, a construction method for the pZE-tdh-gre2p is as follows: a tdh gene and a gre2p gene are obtained by PCR amplification, and the tdh gene and the gre2p gene are co-ligated to the vector pZElac comprising an IPTG inducible promoter, and transformed to a competent cell; and after sequencing, the plasmid pZE-tdh-gre2p is obtained; a construction method for the pZE-tdh-gre2p is illustratively as follows: a tdh gene and a gre2p gene are separately obtained by PCR amplification by using the genomes of *Escherichia coli* MG1655 and *Saccharomyces cerevisiae* S288C as templates, and the tdh gene and the gre2p gene are co-ligated to the vector pZElac comprising an IPTG inducible promoter, and transformed into an *Escherichia coli* dh5a competent cell; and after sequencing, the plasmid pZE-tdh-gre2p is obtained.

9. The preparation method for isopropanolamine as claimed in claim 7, wherein the microorganism is selected from one or more of *Escherichia coli, Bacillus, Corynebacterium, Saccharomyces,* or *Streptomyces.*

10. The preparation method for isopropanolamine as claimed in claim 7, wherein the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

11. The preparation method for isopropanolamine as claimed in any one of claims 4-10, wherein during the fermentation, a fermentation temperature is 20-90 °C.

12. The preparation method for isopropanolamine as claimed in any one of claims 4-10, wherein during the fermentation, a culture medium used comprises starting materials in the following proportions: 11-13 g/L of M9 salt, 1-5 g/L of magnesium sulfate, 0.1-0.5 g/L of calcium chloride, 0.01-0.05 g/L of thiamine, 10-100 g/L of auxiliary material, 3-8 g/L of yeast powder, 1-3 mM/L of IPTG, and 30-60 µg/mL of ampicillin.

13. A recombinant microorganism for preparing isopropanolamine, wherein the recombinant microorganism comprises an oxidase-encoding gene and a reductase-encoding gene, wherein preferably, the oxidase-encoding gene comprises any one or more of tdh, yiaY, and adhB, and is preferably tdh; more preferably, the nucleotide sequence of the tdh gene is set forth in SEQ ID NO: 1;
and/or, the reductase-encoding gene comprises any one or more of gre2p, egsA, SU7, and VIN7, and is preferably gre2p; more preferably, the nucleotide sequence of the gre2p gene is set forth in SEQ ID NO: 2;
preferably, the microorganism is selected from one or more of *Escherichia coli, Bacillus, Corynebacterium, Saccharomyces,* or *Streptomyces.* More preferably, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

14. A recombinant DNA or biomaterial for preparing isopropanolamine, wherein the recombinant microorganism or biomaterial comprises an oxidase-encoding gene and a reductase-encoding gene, wherein
preferably, the oxidase-encoding gene comprises any one or more of tdh, yiaY, and adhB, and is preferably tdh; more preferably, the nucleotide sequence of the tdh gene is set forth in SEQ ID NO: 1;
and/or, the reductase-encoding gene comprises any one or more of gre2p, egsA, SU7, and VIN7, and is preferably gre2p; more preferably, the nucleotide sequence of the gre2p gene is set forth in SEQ ID NO: 2;
preferably, the biomaterial is an expression cassette, a transposon, a plasmid vector, a phage vector, or a virus vector.

15. Use of the recombinant microorganism as claimed in claim 13 and the recombinant DNA or biomaterial as claimed in claim 14 for manufacturing isopropanolamine.
